# FASCICULE DE BREVET EUROPEEN

(11) **EP 3 527 625 B1**
(45) Date de publication et mention de la délivrance du brevet: **19.08.2020**
(21) Numéro de dépôt: 19158093.5
(22) Date de dépôt: 19.02.2019
(51) Int. Cl.: C08L 83/04, A61K 8/58, A61Q 19/06, A61Q 19/08

(54) **COMPOSITION COMPRENANT DU MONOMETHYLSILANETRIOL**
MONOMETHYLSILANETRIOL ENTHALTENDE ZUSAMMENSETZUNG
COMPOSITION COMPRISING MONOMETHYLSILANETRIOL

(30) Priorité: 19.02.2018 BE 201800023
(43) Date de publication de la demande: 21.08.2019
(73) Titulaire: Soles Mundi Sprl, 6220 Fleurus (BE)
(72) Inventeur: SAMMARTINO, Luca, 4652 Xhendelesse (BE); IANNELLO, Léonard, 4652 Xhendelesse (BE); IANNELLO, Salvatore, 4652 Xhendelesse (BE)
(74) Mandataire: ABYOO

(56) Documents cités:
- EP-A1- 0 281 435
- EP-A1- 0 391 769
- EP-A1- 2 172 186
- CH-A5- 686 997
- FR-A1- 2 897 532
- FR-A1- 3 038 898
- FR-A1- 3 051 672

## Description

### Domaine de l'invention

La présente invention se rapporte à une composition comprenant du monométhylsilanetriol (MMST), c'est-à-dire comprenant du silicium organique. La présente invention se rapporte également à un procédé de fabrication et à une utilisation d'une telle composition.

### État de la technique

Dans la nature, on distingue deux types de silicium, à savoir le silicium minéral et le silicium organique. Le silicium minéral se retrouve essentiellement sous forme amorphe ou cristalline dans de nombreuses roches comme les granites, les sables ou encore les argiles. Le silicium organique se différencie du silicium minéral par la présence d'un ou de plusieurs atome(s) de carbone associé(s) à de l'hydrogène, cette forme organique étant l'un des éléments essentiels de la matière vivante chez les plantes, les animaux mais aussi chez l'homme.

La dénomination de « silicium organique » a également été attribuée au monométhylsilanetriol (MMST) mais cette appellation est strictement d'origine technique, cette molécule étant issue de la chimie organique. Une distinction doit donc être faite entre le silicium organique d'origine naturelle et le silicium organique issu de la chimie organique tel que le MMST. Afin d'éviter toute confusion, la dénomination MMST sera utilisée dans le cadre de la présente invention pour bien indiquer qu'une composition suivant l'invention comprend du silicium organique issu de la chimie organique et non pas du silicium organique d'origine naturelle.

Le MMST, monométhylsilanetriol ou encore 1-méthyl-silanetriol (CH₆O₃Si) diffère de par sa structure du silicium naturel, ce dernier étant retrouvé sous forme d'acide orthosilicique notamment dans l'alimentation. Concrètement, pour le MMST (silicium organique), un groupe méthyle (1 carbone + 3 hydrogènes) remplace un groupe hydroxyde (oxygène + hydrogène), ce qui entraine une modification au niveau énergétique de la molécule dès lors que la liaison C-Si est d'une énergie très élevée. En ce sens, sur base des champs d'énergie calculée, il a été montré que la molécule MMST présente simultanément deux « zones », une première zone comportant des charges négatives, des charges positives et des charges neutres et une seconde zone ne comportant que des charges neutres. La première zone confère à la molécule MMST une polarité variable de telle sorte qu'elle se comporte comme de l'eau tandis que la deuxième zone confère au MMST les propriétés d'un lipide. Par conséquent, la molécule MMST a la capacité de se déplacer tant dans un milieu aqueux que dans un milieu lipidique et peut ainsi être qualifiée de molécule amphiphile (molécule soluble à la fois dans l'eau et dans l'huile).

Cette propriété amphiphile de la molécule MMST lui confère un intérêt considérable puisque les cellules (dont celles du corps humain) se composent d'eau d'une part et de membranes lipidiques d'autre part (autour et dans la cellule). En d'autres termes, la molécule MMST est capable de passer et de se déplacer dans toutes les cellules, ce qui permet d'assurer une libération efficace de silicium dans l'organisme (biodisponibilité). Plusieurs études ont été réalisées afin d'exploiter cette propriété de la molécule MMST notamment pour étudier son effet sur la densité osseuse, sur l'ostéoporose et sur le système immunitaire.

En outre, il est reconnu aujourd'hui que le corps humain est généralement en carence de silicium. Ceci est notamment dû au fait que la quantité de silicium présente dans le corps diminue naturellement avec l'âge : le silicium est en permanence utilisé par le corps dans les tissus ; l'excédent de silicium absorbé quotidiennement n'est pas stocké et, avec le temps, notre organisme est de moins en moins apte à assimiler le silicium issu de notre alimentation. Une autre raison de cette carence en silicium est que l'apport en cet élément via notre nourriture a grandement diminué : appauvrissement des sols en minéraux et donc des aliments issus des cultures ; diminution des apports en micro-nutriments résultant de la transformation des aliments au travers des processus de production industriels.

Pourtant, le corps humain a de plus en plus besoin de silicium, d'autant que nos régimes alimentaires actuels, trop riches en glucides et en protéines animales, participent à l'acidification du corps. En effet, pour assurer le bon fonctionnement de l'organisme, l'excès d'acidité est régulé en soutirant les minéraux des tissus, cette déminéralisation étant à la base de pathologies lourdes comme l'ostéoporose.

Les conséquences négatives d'une carence en silicium sont d'une part la présence de tissus plus fragiles et cassants à cause d'une perte de flexibilité (vieillissement prématuré, peau ridée, articulations enflammées, arthrose, perte de mobilité, ostéoporose, cheveux et ongles fragilisés, troubles de la pression artérielle) et, d'autre part, des pertes de fonctionnalité de l'organisme (augmentation du risque d'inflammation par fragilisation des tissus, stress oxydatif, fatigue, inefficacité digestive, activité cellulaire réduite).

Des compositions comprenant du monométhylsilanetriol (MMST), c'est-à-dire comprenant du silicium organique, sont connues de l'état de la technique, par exemple sous forme liquide ou sous forme de gel.

Malheureusement, dans les compositions actuelles, dès que la concentration en MMST est supérieure à 0,04% (v/v), soit supérieure à 4,1 mM (ce qui correspond à une concentration de 115 mg Si/L), les molécules de MMST subissent une polymérisation en dimères et en trimères, ce qui altère fortement les propriétés et l'efficacité des molécules de MMST. Plus spécifiquement, ce phénomène de polymérisation entraine irrémédiablement un retour du MMST (silicium organique) à sa forme minérale, la propriété amphiphile et tout l'intérêt du MMST étant ainsi perdus. En outre, dans les compositions actuelles, dès que la concentration en MMST est supérieure à 0,04% (v/v), soit supérieure à 4,1 mM (ce qui correspond à une concentration de 115 mg Si/L), un précipité important se forme, ce qui rend la composition impropre à la consommation en plus d'altérer les propriétés et l'efficacité des molécules de MMST. Actuellement, il n'existe donc pas de composition comprenant du MMST à une concentration supérieure à 0,04% (v/v), soit supérieure à 4,1 mM, ce qui limite fortement l'utilisation de cette molécule d'intérêt.

Le document FR 3 051 672 décrit des compositions qui comprennent du monométhyltrisilanol, un agent complexant ainsi que différents composés additionnels tels que des falvonoïdes. La composition est destinée à un usage cosmétique.

### Résumé de l'invention

Un des buts de la présente invention est de procurer une composition pouvant comprendre du monométhylsilanetriol (MMST) à une concentration supérieure à 0,04% (v/v), soit supérieure à 4,1 mM (ce qui correspond à une concentration de 115 mg Si/L) en assurant que le MMST reste sous sa forme organique sans qu'il n'y ait polymérisation du MMST et sans qu'un précipité ne se forme, de telle sorte que la composition est stable et reste consommable au cours du temps.

L'invention est définie par les revendications indépendantes. Les revendications dépendantes définissent des modes de réalisation préférés de l'invention.

Pour résoudre au moins partiellement les problèmes rencontrés avec les compositions actuelles comprenant du MMST, il est prévu, suivant la présente invention, une composition comprenant du monométhylsilanetriol (MMST) et un agent complexant, ladite composition étant caractérisée en ce que ledit agent complexant est du glycérophosphate de magnésium et en ce que ledit monométhylsilanetriol et ledit agent complexant y sont présents selon un ratio d'équivalence molaire monométhylsilanetriol : agent complexant compris entre 1 : 0,025 et 1 : 10, de préférence compris entre 1 : 0,05 et 1 : 5.

Au sens de l'invention, un agent complexant (ou une molécule complexante), possède des sites complexants permettant de fixer au moins un ion métallique, en particulier permettant de fixer au moins un des ions métalliques suivants : Al³⁺, Ag⁺, Ca²⁺, Cu⁺, Cu²⁺, Fe²⁺, Fe³⁺, K⁺, Mg²⁺, Na⁺ et Zn²⁺. Un tel agent complexant correspond à un ligand, lequel est un atome, un ion ou une molécule portant des fonctions chimiques lui permettant de se lier à un ou plusieurs atomes ou ions centraux.

Lorsque ledit agent complexant (molécule complexante) fixe au moins un ion métallique, peut être formé un complexe ou un chélate. Un « chélate » se distingue d'un « simple complexe » par le fait que le cation métallique est fixé à un ligand chélateur par au moins deux liaisons de coordination : on parle ainsi de « complexe de coordination » dans le cas d'un chélate. Dans le cas d'un chélate, ledit agent complexant (molécule complexante) possède donc au moins deux sites complexants.

Dans le cadre de la présente invention, il a été mis en évidence qu'une telle composition comprenant du MMST et du glycérophosphate de magnésium comme agent complexant selon un ratio d'équivalence molaire monométhylsilanetriol : agent complexant compris entre 1 : 0,025 et 1 : 10, de préférence compris entre 1 : 0,05 et 1 : 5 n'est ni sujette à la problématique de formation d'un précipité important, ni sujette à la problématique de polymérisation du MMST en dimères et/ou en trimères même pour une concentration en MMST supérieure à 0,04% (v/v), soit supérieure à 4,1 mM. Plus particulièrement, il a été mis en évidence qu'un tel ratio d'équivalence molaire monométhylsilanetriol : agent complexant (glycérophosphate de magnésium) compris entre 1 : 0,025 et 1 : 10, de préférence compris entre 1 : 0,05 et 1 : 5 dans une composition selon l'invention permet d'obstruer cette dernière de telle sorte que les molécules de MMST n'ont pas la possibilité de polymériser. Selon l'invention, cette obstruction de la composition est réalisée sans formation de complexes stables ou de chélates stables entre les molécules de MMST et l'agent complexant (glycérophosphate de magnésium) au sein de la composition, ceci garantissant que le MMST conserve toute son efficacité en restant disponible. En d'autres termes, il a été déterminé, dans le cadre de la présente invention, qu'un tel ratio molaire est optimal et qu'il permet d'assurer que la composition comprenant du MMST et au moins un agent complexant (glycérophosphate de magnésium) est stable, ne donne pas lieu à la formation d'un précipité important et qu'elle n'est pas sujette à la problématique de polymérisation du MMST en dimères et/ou en trimères même pour une concentration en MMST supérieure à 0,04% (v/v), soit supérieure à 4,1 mM. Par ailleurs, un tel ratio assure que l'obstruction de la composition s'effectue sans formation aucune de complexes stables qui diminuerait l'efficacité et la disponibilité du MMST.

Une composition selon l'invention reste donc stable et consommable au cours du temps sans polymérisation des molécules de MMST et sans formation d'un précipité.

Par les termes « obstruer » et « obstruction » de la composition, il est entendu, au sens de la présente invention, que l'agent complexant présent empêche les molécules de MMST de réagir entre elles pour former des molécules de masses molaires plus élevées, c'est-à-dire de polymériser en dimères et en trimères, voire en de plus longues chaines moléculaires.

Il a par ailleurs été observé qu'une composition selon l'invention, dans laquelle ledit monométhylsilanetriol et ledit glycérophosphate de magnésium comme agent complexant sont présents selon un ratio d'équivalence molaire monométhylsilanetriol : agent complexant compris entre 1 : 0,025 et 1 : 10, de préférence compris entre 1 : 0,05 et 1 : 5, permet d'assurer que le MMST conserve bien ses propriétés amphiphiles et son efficacité tout en pouvant être présent en une concentration supérieure à 0,04% (v/v) qui permet d'assurer en final une concentration en silicium (Si) supérieure à 115 mg/L dans les compositions selon l'invention. Ceci est particulièrement avantageux puisque, à volumes ou à poids égaux, des quantités plus importantes de MMST et donc de silicium (Si) peuvent être administrées avec une composition selon l'invention par rapport aux compositions de l'état de la technique.

Avantageusement, selon l'invention, ledit monométhylsilanetriol et ledit agent complexant sont présents dans la composition selon un ratio d'équivalence molaire monométhylsilanetriol : agent complexant compris entre 1 : 0,05 et 1 : 20, de préférence compris entre 1 : 0,1 et 1 : 15. De façon plus préférentielle encore, ledit ratio d'équivalence molaire monométhylsilanetriol : agent complexant est compris entre 1 : 0,1 et 1 : 1,5.

Préférentiellement, la composition selon l'invention présente un pH compris entre 6 et 7, de préférence un pH égal à 6,6.

De préférence, la composition selon l'invention comprend en outre au moins un additif et/ou au moins un excipient biocompatible choisi dans le groupe constitué des antioxydants, des agents conservateurs, des vitamines, des acides aminés, des minéraux, des oligo-éléments, des extraits végétaux, des agents stabilisateurs de pH et de leurs mélanges.

Par exemple, comme antioxydants, peuvent être utilisés les composés suivants : les orthophosphates de sodium monosodique, disodique et trisodique; les orthophosphates de potassium monopotassique, dipotassique et tripotassique; les vitamines C et E; l'ascorbate de sodium, l'ascorbate de potassium, l'ascorbate de calcium; le lactate de sodium, le lactate de potassium ou encore le lactate de calcium.

Parmi les agents conservateurs, peuvent être utilisés les composés suivants : l'acide acétique, le diacétate de potassium, l'acétate de potassium; le diacétate de sodium, l'acétate de sodium; l'acétate de calcium; le dioxyde de carbone; l'acide sorbique; le sorbate de potassium, le sorbate de sodium, le sorbate de potassium; les benzoates de sodium, de potassium et de calcium; la nisine ; les sulfites de sodium, de potassium et de calcium ; les disulfited de sodium et de potassium; le sulfite acide de calcium et potassium, les huiles essentielles et les flavonoïdes.

Parmi les agents stabilisateurs de pH, peuvent être utilisés les composés suivants : l'acide lactique, le lactate de magnésium, l'acide malique et ses dérivés, l'acide citrique et ses dérivés, l'acide tartrique et ses dérivés ; les carbonates de sodium, de potassium, d'ammonium et de magnésium; l'acide hydrochlorique, l'acide phosphorique; les hydroxydes de sodium, de potassium, de calcium, d'ammonium et de magnésium.

Des agents épaississants peuvent aussi être intégrés dans une composition selon l'invention comme par exemple l'acide alginique et ses dérivés, la pectine, le glysérophosphate de calcium et la gélatine.

Parmi les oligoéléments, peuvent être utilisés les composés suivants : le sélénium, le potassium, le calcium, le sodium, le magnésium, le cuivre, le fer ou encore le zinc.

Avantageusement, la composition selon l'invention comprend en outre au moins un flavonoïde d'origine naturelle ou non, en particulier au moins un flavonoïde en phase alcoolique ou glycérique. Par exemple, ledit flavonoïde est un extrait d'un végétal de genre Citrus (citron, orange, pamplemousse, ...), avantageusement un extrait d'un végétal de genre Citrus sous forme d'une huile essentielle. Un tel flavonoïde présente des propriétés antifongiques et antibactériennes.

De façon préférée, la composition selon l'invention se présente sous forme liquide ou semi-liquide vaporisable ou non, sous forme d'un gel, d'une boisson ou d'une compresse, sous forme d'une gélule, d'un comprimé ou d'une capsule ou sous toute autre forme adéquate.

La présente invention porte également sur un procédé de fabrication d'une composition suivant l'invention, ledit procédé de fabrication comprenant les étapes suivantes :
- une amenée d'une quantité prédéterminée de monométhylsilanetriol dans un mélangeur,
- une amenée d'une quantité prédéterminée de glycérophosphate de magnésium comme agent complexant dans ledit mélangeur, et
- un mélange, dans ledit mélangeur, dudit monométhylsilanetriol et dudit glycérophosphate de magnésium comme agent complexant,
lesdites étapes d'amenée d'une quantité prédéterminée de monométhylsilanetriol et d'une quantité prédéterminée de glycérophosphate de magnésium comme agent complexant dans ledit mélangeur mettant en œuvre ledit monométhylsilanetriol et ledit glycérophosphate de magnésium comme agent complexant selon un ratio d'équivalence molaire monométhylsilanetriol : agent complexant compris entre 1 : 0,025 et 1 : 10, de préférence compris entre 1 : 0,05 et 1 : 5.

De façon plus préférentielle encore, ledit ratio d'équivalence molaire monométhylsilanetriol : agent complexant est compris entre 1 : 0,05 et 1 : 20 ou entre 1 : 0,1 et 1 : 15 ou entre 1 : 0,1 et 1 : 1,5.

Avantageusement, selon l'invention, ladite étape d'amenée d'une quantité prédéterminée de monométhylsilanetriol dans le mélangeur est précédée par une étape d'obtention dudit monométhylsilanetriol par mise en solution de méthylsiliconate de sodium et/ou de méthylsiliconate de potassium dans un milieu acide présentant une valeur de pH inférieur à 7 et de préférence une valeur de pH comprise entre 2 et 5. Eventuellement, selon l'invention, ladite étape d'amenée d'une quantité prédéterminée de monométhylsilanetriol est remplacée par ladite mise en solution, dans ledit mélangeur, de méthylsiliconate de sodium et/ou de méthylsiliconate de potassium dans ledit milieu acide.

La présente invention porte également sur une utilisation d'une composition suivant l'invention, pour la fabrication de préparations ou de compléments alimentaires, diététiques, cosmétiques et pharmaceutiques à usage humain ou animal.

### Exemple

Afin d'évaluer la stabilité au cours du temps de compositions selon l'invention, différentes compositions comprenant du monométhylsilanetriol (MMST) et du glycérophophate de magnésium en tant qu'agent complexant ont été formulées selon les ratios d'équivalence molaire monométhylsilanetriol : agent complexant indiqués dans le Tableau 1 ci-dessous.

Chacune de ces compositions a été formulée en suivant le protocole suivant :
1) Dissolution de 0,52 g d'acide citrique dans 1L d'eau osmosée;
2) Ajout, dans la solution obtenue à l'étape 1, de la quantité adéquate de glycérophophate de magnésium pour obtenir un ratio molaire monométhylsilanetriol : agent complexant donné;
3) Ajout, dans la solution obtenue à l'étape 2, de 0,581 g de MMST de telle façon à obtenir une solution où la concentration en MMST est de 6,2 mM;
4) Ajustement du pH de la solution obtenue à l'étape 3 par ajout d'acide phosphorique pour atteindre un pH de 6,6.

Chacune des solutions a ensuite été soumise a un vieillissement forcé dans une étuve à 40°C durant 2 mois. Un tel vieillissement forcé à 40°C correspond à un vieillissement de facteur 4, ce qui signifie que les résultats observés après 2 mois correspondent à un temps de vieillissement de 8 mois. Suite au vieillissement forcé, afin de juger de la stabilité des compositions selon l'invention présentant des ratios d'équivalence molaire monométhylsilanetriol : agent complexant différents, il a été observé visuellement si les compositions étaient toujours transparentes et si la formation d'un précipité avait eu lieu ou non. Les résultats obtenus sont présentés dans le Tableau 1.

### Tableau 1

| | |
|---|---|
| **Ratio d'équivalence molaire MMST:agent complexant** | **Observations** |
| 1 : 0 | Instable / Impropre à la consommation |
| | Présence d'un précipité blanchâtre |
| 1 : 0,025 | Stable / Consommable |
| | Très faible présence d'un précipité |
| 1 : 0,05 | Stable / Consommable |
| 1 : 0,1 | Stable / Consommable |
| 1 : 0,2 | Stable / Consommable |
| 1 : 0,5 | Stable / Consommable |
| 1 : 1 | Stable / Consommable |
| 1 : 1,5 | Stable / Consommable |
| 1 : 2 | Stable / Consommable |
| 1 : 5 | Stable / Consommable |
| 1 : 10 | Stable / Consommable |
| | Très faible présence d'un précipité |
| 1 : 15 | Instable mais consommable |
| | Faible présence d'un précipité |
| 1 : 20 | Instable / Impropre à la consommation |
| | Forte présence d'un précipité blanchâtre |
| 1 : 50 | Instable / Impropre à la consommation |
| | Forte présence d'un précipité blanchâtre |
| | Polymérisation du MMST |

Comme on peut le constater, lorsque ledit monométhylsilanetriol et ledit au moins un agent complexant sont présents selon un ratio d'équivalence molaire monométhylsilanetriol : agent complexant compris entre 1 : 0,025 et 1 : 10, de préférence compris entre 1 : 0,05 et 1 : 5, dans une composition selon l'invention, celle-ci reste stable et consommable au-delà de 6 mois de vieillissement, la composition comprenant du monométhylsilanetriol (MMST) à une concentration supérieure à 0,04% (v/v), soit supérieure à 4,1 mM (ce qui correspond à une concentration de 115 mg Si/L). Par contre, lorsque le ratio d'équivalence molaire monométhylsilanetriol : agent complexant est inférieur à 1 : 0,025 ou lorsque le ratio d'équivalence molaire monométhylsilanetriol : agent complexant est supérieur à 1 : 10, les compositions ne sont pas stables et deviennent impropres à la consommation (formation d'un précipité et/ou polymérisation du MMST).

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition comprenant du monométhylsilanetriol et un agent complexant, ladite composition étant **caractérisée en ce que** ledit agent complexant est du glycérophosphate de magnésium et **en ce que** ledit monométhylsilanetriol et ledit agent complexant y sont présents selon un ratio d'équivalence molaire monométhylsilanetriol : agent complexant compris entre 1 : 0,025 et 1 : 10.

2. Composition selon la revendication 1, **caractérisée en ce qu'**elle présente un pH compris entre 6 et 7, de préférence un pH égal à 6,6.

3. Composition selon l'une quelconque des revendications précédentes, **caractérisée en ce qu'**elle comprend en outre au moins un additif et/ou au moins un excipient biocompatible choisi dans le groupe constitué des antioxydants, des agents conservateurs, des vitamines, des minéraux, des oligo-éléments, des extraits végétaux, des agents stabilisateurs de pH et de leurs mélanges.

4. Composition selon l'une quelconque des revendications précédentes, **caractérisée en ce qu'**elle comprend en outre au moins un flavonoïde d'origine naturelle ou non, en particulier au moins un flavonoïde en phase alcoolique ou glycérique.

5. Composition selon l'une quelconque des revendications précédentes, **caractérisée en ce qu'**elle se présente sous forme liquide ou semi-liquide vaporisable ou non, sous forme d'un gel, d'une boisson ou d'une compresse, sous forme d'une gélule, d'un comprimé ou d'une capsule.

6. Procédé de fabrication d'une composition selon l'une quelconque des revendications 1 à 5, ledit procédé comprenant les étapes suivantes :
- une amenée d'une quantité prédéterminée de monométhylsilanetriol dans un mélangeur,
- une amenée d'une quantité prédéterminée de glycérophosphate de magnésium comme agent complexant dans ledit mélangeur, et
- un mélange, dans ledit mélangeur, dudit monométhylsilanetriol et dudit de glycérophosphate de magnésium comme agent complexant,
lesdites étapes d'amenée d'une quantité prédéterminée de monométhylsilanetriol et d'une quantité prédéterminée de glycérophosphate de magnésium comme agent complexant dans ledit mélangeur mettant en œuvre ledit monométhylsilanetriol et ledit glycérophosphate de magnésium comme agent complexant selon un ratio d'équivalence molaire monométhylsilanetriol : agent complexant compris entre 1 : 0,025 et 1 : 10.

7. Procédé de fabrication selon la revendication 6, caractérisé en ce ladite étape d'amenée d'une quantité prédéterminée de monométhylsilanetriol dans le mélangeur est précédée par une étape d'obtention dudit monométhylsilanetriol par mise en solution de méthylsiliconate de sodium et/ou de méthylsiliconate de potassium dans un milieu acide présentant une valeur de pH inférieur à 7 et de préférence une valeur de pH comprise entre 2 et 5.

8. Procédé de fabrication selon la revendication 6 ou 7, **caractérisé en ce qu'**il comprend en outre une étape additionnelle d'amenée d'au moins un excipient biocompatible avant ou pendant ladite étape de mélange.

9. Utilisation d'une composition selon l'une quelconque des revendications 1 à 5, pour la fabrication de préparations ou de compléments alimentaires, diététiques, cosmétiques et pharmaceutiques à usage humain ou animal.

## Patentansprüche

1. Zusammensetzung, umfassend Monomethylsilantriol und einen komplexierenden Wirkstoff, wobei die genanntee Zusammensetzung **dadurch gekennzeichnet ist, dass** der genannt komplexierende Wirkstoff Magnesium-Glycerophosphat ist und dass das genannte Monomethylsilantriol und der genannte komplexierende Wirkstoff darin gemäß einem molaren Äquivalenzverhältnis Monomethylsilantriol: komplexierender Wirkstoff, das zwischen 1: 0,025 und 1: 10 inbegriffen ist, vorhanden sind.

2. Zusammensetzung gemäß Anspruch 1, **dadurch gekennzeichnet, dass** sie einen zwischen 6 und 7 inbegriffenen pH-Wert, bevorzugt einen pH-Wert gleich 6,6 aufweist.

3. Zusammensetzung gemäß irgendeinem der voranstehenden Ansprüche, **dadurch gekennzeichnet, dass** sie darüber hinaus wenigstens einen Zusatz und / oder wenigstens einen biokompatiblen Hilfsstoff umfasst, der aus der Gruppe ausgewählt ist, die aus Antioxidantien, Konservierungsstoffen, Vitaminen, Mineralien, Spurenelementen, Pflanzenextrakten, Stabilisatoren des pH-Wertes und deren Mischungen gebildet ist.

4. Zusammensetzung gemäß irgendeinem der voranstehenden Ansprüche, **dadurch gekennzeichnet, dass** sie darüber hinaus wenigstens ein Flavonoid natürlichen oder nicht-natürlichen Ursprungs, insbesondere wenigstens ein Flavonoid in alkoholischer oder Glycerinphase, umfasst.

5. Zusammensetzung gemäß irgendeinem der voranstehenden Ansprüche, **dadurch gekennzeichnet, dass** sie eine flüssige oder halbflüssige, versprühbare oder nicht-versprühbare Form, die Form eines Gels, eines Getränks oder einer Kompresse, die Form einer Kapsel, einer Tablette oder Pastille aufweist.

6. Herstellungsverfahren einer Zusammensetzung gemäß irgendeinem der Ansprüche 1 bis 5, wobei das genannte Verfahren die folgenden Schritte umfasst:
- eine Zuführung einer vorbestimmten Menge an Monomethylsilantriol in einem Mischer,
- eine Zuführung einer vorbestimmten Menge an Magnesium-Glycerophosphat als komplexierenden Wirkstoff in dem genannten Mischer und
- eine Mischung des genannten Monomethylsilantriols und des genannten Magnesium-Glycerophosphats als komplexierender Wirkstoff in dem genannten Mischer,
wobei die genannten Zuführschritte einer vorbestimmten Menge an Monomethylsilantriol und einer vorbestimmten Menge an Magnesium-Glycerophosphat als komplexierender Wirkstoff in dem genannten Mischer das genannte Monomethylsilantriol und das genannte Magnesium-Glycerophosphat als komplexierenden Wirkstoff gemäß einem molaren Äquivalenzverhältnis Monomethylsilantriol: komplexierender Wirkstoff, das zwischen 1: 0,025 und 1: 10 inbegriffen ist, umsetzt.

7. Herstellungsverfahren gemäß Anspruch 6, **dadurch gekennzeichnet, dass** dem genannten Zuführschritt einer vorbestimmten Menge an Monomethylsilantriol in dem Mischer ein Schritt zum Erhalten des genannten Monomethylsilantriols per Lösen von Natrium-Methylsilikonat und / oder KaliumMethylsilikonat in einem sauren Milieu vorangeht, das einen pH-Wert von unter 7 und bevorzugt einen zwischen 2 und 5 inbegriffenen pH-Wert aufweist.

8. Herstellungsverfahren gemäß Anspruch 6 oder 7, **dadurch gekennzeichnet, dass** es darüber hinaus einen zusätzlichen Zuführschritt wenigstens eines biokompatiblen Hilfsstoffs vor oder während des genannten Mischschritts umfasst.

9. Verwendung einer Zusammensetzung gemäß irgendeinem der Ansprüche 1 bis 5 für die Herstellung von Zubereitungen oder Nahrungs-, diätetischen, kosmetischen und pharmazeutischen Ergänzungsmitteln für den Einsatz beim Menschen oder Tier.

## Claims

1. Composition comprising monomethylsilanetriol and a complexing agent, said composition being **characterised in that** said complexing agent is magnesium glycerophosphate and **in that** said monomethylsilanetriol and said complexing agent are present therein in a molar equivalence ratio of monomethylsilanetriol : complexing agent of between 1 : 0.025 and 1 : 10.

2. Composition according to claim 1, **characterised in that** it has a pH between 6 and 7, preferably a pH equal to 6.6.

3. Composition according to any of the preceding claims, **characterised in that** it additionally comprises at least one biocompatible excipient and/or at least one additive selected from a group consisting of antioxidants, preservatives, vitamins, minerals, trace elements, plant extracts, pH stabilising agents and mixtures thereof.

4. Composition according to any one of the preceding claims, **characterised in that** it additionally comprises at least one flavonoid of natural or unnatural origin, in particular at least one flavonoid in the alcoholic or glyceric phase.

5. Composition according to any one of the preceding claims, **characterised in that** it is in liquid or semi-liquid form, vaporisable or not, in the form of a gel, a drink or a compress, in the form of a capsule, a tablet or a capsule.

6. Method of manufacturing a composition according to any one of claims 1 to 5, said method comprising the following steps:
- adding a predetermined amount of monomethylsilanetriol into a mixer,
- adding a predetermined amount of magnesium glycerophosphate as a complexing agent into said mixer, and
- a mixture, in said mixer, of said monomethylsilanetriol and magnesium glycerophosphate as a complexing agent,
said steps of adding a predetermined amount of monomethylsilanetriol and a predetermined amount of magnesium glycerophosphate as complexing agent into said mixer using said monomethylsilanetriol and said magnesium glycerophosphate as complexing agent at a molar equivalence ratio of monomethylsilanetriol : complexing agent of between 1 : 0.025 and 1 : 10.

7. Method of manufacturing according to claim 6, **characterised in that** the said step of adding a predetermined quantity of monomethylsilanetriol into the mixer is preceded by a step of obtaining the said monomethylsilanetriol by dissolving sodium methylsiliconate and/or potassium methylsiliconate in an acid medium having a pH value of less than 7 and preferably a pH value of between 2 and 5.

8. Method of manufacturing according to claim 6 or 7, **characterised in that** it further comprises an additional step of adding at least one biocompatible excipient before or during said mixing step.

9. Use of a composition according to any of claims 1 to 5, for the manufacture of food, dietetic products, cosmetics and pharmaceutical preparations or supplements for human or animal use.
